# EUROPEAN PATENT SPECIFICATION

(11) **EP 2 729 187 B1**
(45) Date of publication and mention of the grant of the patent: **26.08.2020**
(21) Application number: 12740921.7
(22) Date of filing: 09.07.2012
(51) Int. Cl.: A61L 2/22, A01N 25/06, A01N 27/00, A01N 65/00, A01N 65/08, A01N 65/22, A01N 65/28, B05B 1/26, B05B 7/12, B05B 7/24, B05B 1/14

(54) **DISINFECTING METHOD FOR DISINFECTING A ROOM OR SURFACE, AND DISINFECTING FLUID COMPOSITION SUITABLE FOR TRANSFORMING INTO AN AEROSOL OF FLUID PARTICLES SUSPENDED IN A GAS**
DESINFEKTIONSVERFAHREN ZUR DESINFEKTION EINES RAUMES ODER EINER OBERFLÄCHE SOWIE DESINFEKTIONSFLÜSSIGKEITSZUSAMMENSETZUNG ZUR UMWANDLUNG VON IN EINEM GAS SUSPENDIERTEN FLÜSSIGEN PARTIKELN IN EIN AEROSOL
PROCÉDÉ DE DÉSINFECTION POUR LA DÉSINFECTION D'UNE PIÈCE OU D'UNE SURFACE, ET COMPOSITION FLUIDE DÉSINFECTANTE SE PRÊTANT À ÊTRE TRANSFORMÉE EN AÉROSOL DE PARTICULES DE FLUIDE EN SUSPENSION DANS UN GAZ

(30) Priority: 08.07.2011 NL 2007071
(43) Date of publication of application: 14.05.2014
(73) Proprietor: Zarfl, Hans Peter, 9400 Wolfsberg (AT)
(72) Inventor: Zarfl, Hans Peter, 9400 Wolfsberg (AT)
(74) Representative: KLIMENT & HENHAPEL
(86) International application number: PCT/EP2012/063401
(87) International publication number: WO 2013/007688

(56) References cited:
- WO-A1-98/21307
- WO-A1-2009/124392
- CH-A5- 688 787
- CN-A- 101 406 177
- JP-A- 2007 100 059
- US-A- 3 954 974
- US-A1- 2002 068 101
- US-A1- 2004 009 094
- US-A1- 2009 148 342
- US-A1- 2010 034 907
- AGGAG M E ET AL: "STUDY OF ANTI MICROBIAL ACTIVITY OF CHAMOMILE OIL", PLANTA MEDICA, THIEME VERLAG, DE, vol. 22, no. 2, 1 January 1972 (1972-01-01), pages 140-144, XP009165754, ISSN: 0032-0943
- Albert E Ebert: "The standard formulary (page 171)", , 1 January 1897 (1897-01-01), page 171, XP055620269, Chicago Retrieved from the Internet: URL:https://books.google.com/ [retrieved on 2019-09-10]

## Description

The present invention is related to a disinfecting method for disinfecting a room or a surface, wherein a flow of an aerosol is used as a disinfectant.

A disinfecting method is in general aimed at inactivating microorganisms, for sanitary reasons.

It is further known to use an aerosol as a disinfectant and to apply a flow of aerosol through a room or onto a surface that should be disinfected. Thus, a room containing airborne microorganisms can be sanitized. Alternatively, a surface on which microorganisms are present can be sanitized. In both ways, any confined space designed for humans or animals can be sanitized in order to reduce or eliminate the risk of infection by microorganisms.

Most known disinfectants are however potentially harmful to humans or animals, and require a careful use thereof as well as additional safety measures before a sanitized space is used by humans or animals. This drawback is even more prominent, when the disinfectant is used in the form of an aerosol, because any unwanted absorption by a living creature (through the skin or through the respiratory tract) is more prone to occur, when compared to a disinfectant that is used in liquid form.

Furthermore, using an aerosol for disinfecting purposes has been found to be less effective in case the suspended particles in the aerosol have a particle size with a broad size distribution, i.e. the particles having a large variation in particle size.

One example for a disinfectant which can be applied by spraying is shown in CH 688787 A5. The disclosed disinfectant may contain natural substances, specifically etheric oils, in order not to have any adverse effects on humans and animals.

WO 2009/124392 A1 relates to an aqueous disinfectant formulation comprising phenolic compounds of natural origin such as thymol, a surfactant, a solvent, and some water; the disinfectant formulation may also be formulated into an aerosol formulation.

US 2009/0148342 A1 discloses a disinfecting fluid based on hypohalous acid. The disinfecting fluid may contain essential oils that exhibit anti-microbial activity and are based on thyme or clove for example. Furthermore, the disinfecting fluid may contain water and calcium hydroxide as buffer or pH adjusting agent.

JP 2007 100059 A refers to a method for producing a cleaning composition containing a natural essential oil encapsulated in solid calcium hydroxide.

Albert E Ebert, The standard formulary, page 171, 1897-01-01, XP055620269, Chicago refers the treatment of burnings by applying absorbant gauze saturated with a liniment comprising linseed oil, lime water and thymol.

The invention is aimed at reducing or eliminating the above drawbacks.

According to the invention the above-mentioned problems are solved by a disinfecting method according to claim 1 and by an disinfecting aerosol according to claim 9.

In addition, an attractive way of disinfecting confined spaces in which animals are kept, could significantly reduce the need for treating animals with antibiotics.

Further, the disinfected method is also aimed at sanitizing rooms which are used by humans, such as hospital rooms, the interior of aeroplanes, etc.

Therefore, the invention relates, according to a first aspect thereof, to a disinfecting method for disinfecting a room or a surface, comprising the steps of:
a) providing a fluid comprising at least one organic compound obtainable from natural substances, wherein the organic compound is provided in an non-polar or polar medium, or in a mixture of non-polar and polar media;
b) mixing the fluid with a gas such that finely divided particles of fluid are suspended in the gas, and consequently an aerosol of the suspended fluid particles is formed;
c) directing a flow of the aerosol formed in step b) on said surface or into said room.

The above method is advantageous over the prior art, because it makes use of an organic compound obtainable from natural substances, which compound has a reduced toxicity compared to known disinfectants, while at the same time being effective as a disinfectant when applied in the form of an aerosol. The organic compounds in the form of suspended particles of an aerosol are much better characterized than particles of these organic compounds used in traditional disinfection methods that are, for example, based on vaporisation of these organic substances. Thus, the wished (disinfection) effect can be induced much better and, particularly, in a quantitative way. Hence, if the disinfection method according to the present invention is used to disinfect confined spaces in which animals are kept, especially stables, the need for treating animals with antibiotics can significantly be reduced and, in turn, problems with residues of such antibiotics can be diminished or even avoided.

The non-polar medium can be an oil, preferably olive oil, and the polar medium is water wherein the water contains dissolved calcium hydroxide, i.e. wherein the water is lime water. Other useful dissolved salts are sea salts and sulphur salts.

Preferably, the overall ratio in the fluid between the total of non-polar compounds and the total of polar compounds, is between 4:1 to 1:4, more preferably between 2:1 to 1:2. Typically, the aerosol used in the disinfecting method could be based on a fluid content of 0.1 to 15 ml fluid per cubic meter gas; for instance a fluid content of 1.0 to 3 ml fluid per cubic meter gas is used in the method.

In the method of the invention and subsequent to step b) and prior to step c), the fluid particles of the aerosol of which the mean particle size is 1 micrometer or above, are separated from the aerosol.

The aerosol formed in step b) contains fluid particles having a broad size distribution which ranges from some nanometers to several micrometers. It has been found that in step c), wherein the aerosol is applied as a disinfectant, it is advantageous to apply an aerosol which contains particles having a mean particle size below 1 micrometer. Especially the range of a mean particle size between 10 and 100 nanometer was found to improve the effectiveness of the aerosol as a disinfectant.

Any known separation method for the aerosol may be applied, such as filtration. Another separation method, is based on the insight that when leading a flow of aerosol over deflecting surfaces, the larger particles (i.e. larger than 1 micrometer) could be separated from the smaller particles.

Separating the larger particles makes the size distribution of the remaining smaller particles narrower - the aerosol is better characterized. This in turn reduces irritation effects that might occur when less-characterized particles with a larger size distribution are used for disinfection, particularly for disinfection of confined spaces in which animals are kept. I.e. in the latter case, the well-characterized aerosol with the narrow size distribution reduces possible irritations of the animals.

Another advantage of the narrow size distribution is that deposited masses can be calculated very accurately. This enables one to precisely use only the necessary - and thus very limited - amount of particles for disinfection. In case of disinfection of the air in confined spaces in which animals are kept, especially in case of disinfection of the air in stables, the limited amount of particles further helps to avoid irritations of the animals.

Advantageously, in the method of the invention, the separated fluid particles of the aerosol are returned to the fluid that is used in step b). As such, the fluid particles which are too large for the disinfecting method, are recycled so that no fluid is lost in the process.

In step a) of the disinfecting method of the invention, the at least one organic compound is chosen from the group consisting of: azulene, the essential oil of thyme, the essential oil of cloves, a resin from the tree *Boswellia,* derivates of the above organic compounds, and mixtures of the above organic compounds.

Azulene is a compound obtained from the distillate of camomile. The essential oils indicated above, refer to the oily substance that can be directly obtained from thyme and cloves, either by extraction or distillation processes. The resin from plants is meant as the viscous substance that is obtained from the stems of plants. A particular useful resin for the invention is Frankincense (also referred to as Olibanum) which is the aromatic resin obtained from the tree *Boswellia.*

With special preference, in step a) of the disinfecting method of the invention,
- the at least one organic compound is provided in a mixture of a non-polar and a polar medium, wherein
   the non-polar medium is an oil, preferably olive oil.

It was found that when the organic compound is provided in the above type of medium, the disinfecting effect is further enhanced.

Furthermore, in step a) of the disinfecting method of the invention, - the fluid provided comprises an organic compound in water, the organic compound being present in an amount of 5-50 wt.%, preferably 10-30 wt.%, of the fluid.

The above range for the content of organic compound in the fluid, was found to be an effective concentration when used in the disinfecting method. The same favourable range is applicable to a mixture of organic compounds is used, wherein each organic compound is present in an amount of 5-50 wt.%, preferably 10-30 wt.%, of the fluid.

Especially preferred in the disinfecting method of the invention, is to use a mixture of organic compounds in water which comprises azulene, the essential oil of thyme, and the essential oil of cloves.

It was found that when applying an aerosol based on this specific mixture of organic compounds in accordance with the method of the invention, it was possible to achieve a high disinfecting effect. More specifically, it was found possible to reduce the amount of microorganisms that are suspended in the air of a confined room by 80% or more. This reduced amount of microorganisms was measured 36 hours after leading a flow of the aerosol into the confined room according to the invention.

Furthermore, it was found that the disinfecting effect of the method could be further enhanced by including a resin from plants as an organic compound in the fluid composition. A particular useful resin for the invention is Frankincense (also referred to as Olibanum) which is the aromatic resin obtained from the tree *Boswellia.*

According to another preferred variant of the disinfecting method of the invention, in step b), a mixture of the fluid and the gas is led under pressure through a nozzle, so that a spray of aerosol is discharged from the nozzle.

The use of a nozzle to form an aerosol was found both expedient and effective for the method of the invention.

In a specific variant of the disinfecting method of the invention, in step b), the gas is led under pressure through a nozzle and the fluid is admixed to the gas during passage through the nozzle, so that a spray of aerosol is discharged from the nozzle.

Such a method makes use of the Venturi-effect inside the nozzle, wherein the constricted flow of gas through the nozzle has a higher velocity which results in a reduced pressure. The reduced pressure is then effective for drawing in the fluid into the flow of gas, so that the fluid can be admixed to the gas stream, simply by providing a separate conduit for fluid which exits inside the nozzle.

When using a nozzle to form an aerosol according to the method of the invention, it is preferable that subsequent to step b) and prior to step c), the fluid particles of the aerosol of which the mean particle size is 1 micrometer or above, are separated from the aerosol by directing the spray of aerosol into a mixing chamber in which the spray is deflected by deflecting surfaces, and from which mixing chamber the aerosol is discharged via an outlet in order to perform step c).

As already explained above, the deflecting surfaces were found to be highly effective in separating larger particles (having a mean size of 1 micrometer or more) from the aerosol, which enhances the disinfecting efficacy of the method.

In a further variant of the disinfecting method of the invention, the mixing chamber and the nozzle are assembled in the form of a cartridge which further comprises a reservoir for the fluid which is in fluid communication with the nozzle and the mixing chamber. As such, the cartridge combines three functionalities needed in the method of the invention: i) the mixing of fluid and gas into an aerosol, ii) the separation of larger particles from the aerosol, iii) a reservoir for fluid to which separated particles are returned.

In a final variant of the disinfecting method of the invention, the cartridge further comprises an inlet for supplying fluid to the reservoir from a source outside of the cartridge.

As such, a disinfecting method is provided wherein a non-limited amount of fluid can be used. This is advantageous especially when large rooms are to be disinfected, and wherein the reservoir of the cartridge itself may not have the capacity for discharging the required amount of fluid.

According to a second aspect, the invention relates to an aerosol of fluid particles suspended in a gas having a disinfecting fluid composition, comprising:
- at least one organic compound obtainable from naturalsubstances, which organic compound is present in a non-polar or polar medium, or in a mixture of non-polar and polar media,
- and wherein the at least one organic compound is chosen from the group consisting of: azulene (i.e. a distillate from camomile), the essential oil of thyme, the essential oil of cloves, a resin from the tree *Boswellia,* derivates of the above organic compounds, and mixtures of the above organic compounds.

A particular useful resin for the invention is Frankincense (also referred to as Olibanum) which is the aromatic resin obtained from the tree *Boswellia.*

When such a fluid composition is used in the disinfecting method as described above, the same advantages are achieved as already indicated: in particular, a large reduction of microorganisms in a room treated by the method of the invention is achieved.

Preferably, the disinfecting fluid composition of the invention fulfils the condition that:
- the at least one organic compound is provided in a mixture of a non-polar and a polar medium, wherein
   the non-polar medium is an oil, preferably olive oil.

The related advantages have already been indicated above for the method of the invention when it includes the same features.

Furthermore, in the aerosol Z of the invention, the organic compound is present in an amount of 5-50 wt.%, preferably 10-30 wt.%, of the fluid composition; the at least one organic compound is provided in lime water or in a mixture of a non-polar medium and lime water; and the the fluid particles have a mean particle size below 1 micrometer.

When a mixture of organic compounds is used, the same preferred amount is applicable to each organic compound.

With particular preference, the fluid composition comprises a mixture of the organic compounds azulene, the essential oil of thyme, and the essential oil of cloves, and wherein each organic compound is present in an amount of 5-50 wt.%, preferably 10-30 wt.%, of the fluid composition.

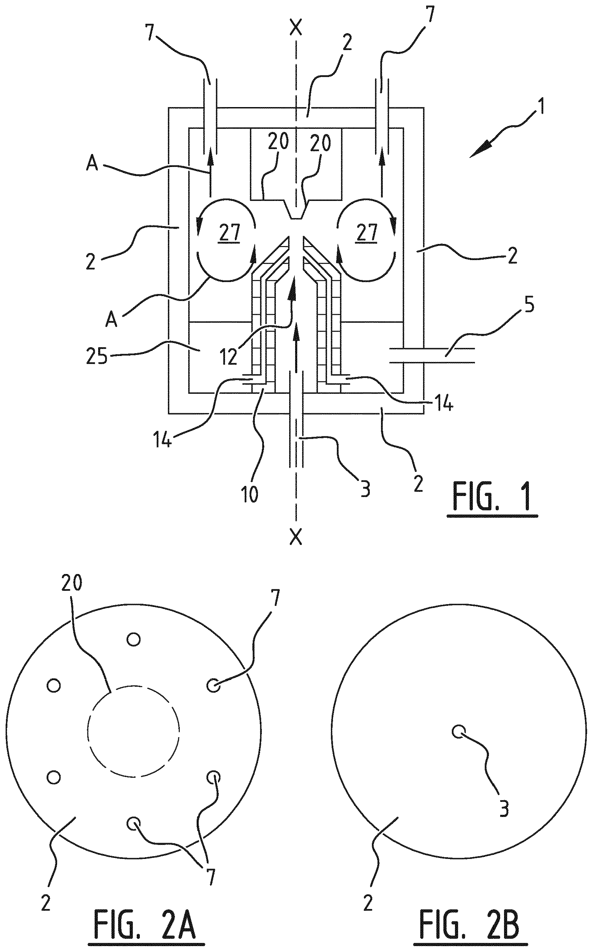

The invention will be further explained according to the example below, together with the appended figures 1 and 2.

### Example

Figure 1 shows a simplified view in cross-section of a cartridge to be used in the method of the invention. Figure 1 shows a cartridge 1 for disinfecting fluid, which has outside walls 2, which are penetrated by: an inlet 3 for pressurized gas from an outside source (not depicted), an inlet 5 for disinfecting fluid from an outside source (not depicted), and outlets 7 for discharging aerosol formed inside the cartridge 1. The cartridge 1 is of a general cylindrical shape, with the dotted line X marking the respective cylindrical axis. The bold arrows A mark the direction of streams of gas and aerosol within the cartridge 1.

Inside the cartridge 1, a conduit 10 for leading the introduced gas is provided, which narrows into a nozzle 12. The conduit 12 has internal channels 14 for leading fluid towards the nozzle 12. The channels 14 are in fluid communication with the narrow part of the nozzle passage 12, at which the gas passing through will exhibit a reduced pressure (i.e. the Venturi effect). Consequently, the fluid 25 kept in the lower part of the cartridge (i.e. the reservoir for fluid), will be drawn through channels 14 towards the nozzle 12 and mix with the gas, thus discharging a spray which is an aerosol of fluid particles in the gas. Opposite to the exit side of the nozzle 12, is a deflecting structure 20 positioned, in order to deflect the spray discharged from the nozzle 12. Grace to the deflection, a turbulent stream of aerosol is formed (depicted by the bent arrows A, which illustrate a circular flow), wherein larger fluid particles will return to the fluid 25, while smaller fluid particles (having a mean size below 1 micrometer) will remain suspended in the gas. At the same time, the aerosol spreads out over mixing chamber 27, and the smaller particles finally exit the cartridge through outlets 7. The aerosol discharged from the outlets 7 may be directly used for disinfecting a room or a surface by leading the flow of aerosol in an appropriate direction. The inlet 5 is used when an additional amount of disinfecting fluid is required inside the cartridge, so that the capacity for discharging aerosol from the cartridge is not restricted to the size of the reservoir inside.

Figure 2A and 2B show respectively a top and bottom view of the cartridge depicted in figure 1. The same features as depicted in figure 1, are indicated by the same reference numbers.

An example of a useful fluid composition for the disinfecting method of the invention, contains 40 wt.% thyme oil, 50 wt.% lime water, and 10 wt.% of a non-polar mixture, which includes oil of cloves, azulene and Frankincense.

Another example of a particularly useful fluid composition for the disinfecting method of the invention contains 49 wt.% thyme oil, 50 wt.% lime water, and 1 wt.% of azulene (i.e. a distillate from camomile).

## Claims

1. Disinfecting method for disinfecting a room or a surface, comprising the steps of:
a) providing a fluid comprising at least one organic compound obtainable from natural substances, wherein the organic compound is provided in a polar medium or in a mixture of non-polar and polar media;
b) mixing the fluid with a gas such that fluid particles are suspended in the gas, and an aerosol of fluid particles is formed;
c) directing a flow of the aerosol formed in step b) on said surface or into said room;
wherein in step a):
- the at least one organic compound is chosen from the group consisting of: azulene, the essential oil of thyme, the essential oil of cloves, a resin obtained from the tree *Boswellia,* derivates of the above organic compounds, and mixtures of the above organic compounds;
and wherein in step a):
- the fluid provided comprises an organic compound in water, the organic compound being present in an amount of 5-50 wt.%, preferably 10-30 wt.%, of the fluid;
**characterized in that** in step a) the at least one organic compound is provided in lime water or in a mixture of a non-polar medium and lime water and wherein subsequent to step b) and prior to step c), the fluid particles of the aerosol of which the mean particle size is 1 micrometer or above, are separated from the aerosol.

2. Disinfecting method according to claim 1, wherein separated fluid particles of the aerosol are returned to the fluid that is used in step b).

3. Disinfecting method according to any of the preceding claims, wherein in step a):
- the at least one organic compound is provided in a mixture of a non-polar medium and lime water, wherein the non-polar medium is an oil, preferably olive oil.

4. Disinfecting method according to any of the preceding claims, wherein in step b):
- a mixture of the fluid and the gas is led under pressure through a nozzle (12), so that a spray of aerosol is discharged from the nozzle (12).

5. Disinfecting method according to any of claims 1 to 3, wherein in step b) :
- the gas is led under pressure through a nozzle (12) and the fluid is admixed to the gas during passage through the nozzle (12), so that a spray of aerosol is discharged from the nozzle (12).

6. Disinfecting method according to any of the preceding claims 4 or 5, wherein subsequent to step b) and prior to step c), the fluid particles of the aerosol of which the mean particle size is 1 micrometer or above, are separated from the aerosol by directing the spray of aerosol into a mixing chamber (27) in which the spray is deflected by deflecting surfaces, and from which mixing chamber (27) the aerosol is discharged via an outlet (7) in order to perform step c).

7. Disinfecting method according to claim 6, wherein the mixing chamber (27) and the nozzle (12) are assembled in the form of a cartridge (1) which further comprises a reservoir for the fluid which is in fluid communication with the nozzle (12) and the mixing chamber (27).

8. Disinfecting method according to claim 7, wherein the cartridge (1) comprises an inlet (5) for supplying fluid to the reservoir from a source outside of the cartridge (1) .

9. Aerosol of fluid particles suspended in a gas, the fluid particles having a disinfecting fluid composition, comprising:
- at least one organic compound obtainable from natural substances, which organic compound is present in a polar medium or in a mixture of non-polar and polar media,
- and wherein the at least one organic compound is chosen from the group consisting of: azulene, the essential oil of thyme, the essential oil of cloves, a resin obtained from the tree *Boswellia,* derivates of the above organic compounds, and mixtures of the above organic compounds, wherein the organic compound is present in an amount of 5-50 wt.%, preferably 10-30 wt.%, of the fluid composition,
**characterized in that** the at least one organic compound is provided in lime water or in a mixture of a non-polar medium and lime water and wherein the fluid particles have a mean particle size below 1 micrometer.

10. Aerosol according to claim 9, wherein
- the at least one organic compound is provided in a mixture of a non-polar medium and lime water, wherein the non-polar medium is an oil, preferably olive oil.

## Patentansprüche

1. Desinfektionsverfahren zur Desinfektion eines Raumes oder einer Oberfläche, umfassend die folgenden Schritte:
a) Bereitstellen eines Fluids, das wenigstens eine organische Verbindung umfasst, die aus natürlichen Substanzen erhältlich ist, wobei die organische Verbindung in einem polaren Medium oder in einem Gemisch aus nichtpolaren und polaren Medien bereitgestellt wird;
b) Mischen des Fluids mit einem Gas, sodass Fluidpartikel in dem Gas suspendiert werden und ein Aerosol aus Fluidpartikeln gebildet wird;
c) Richten eines Stroms des in Schritt b) gebildeten Aerosols auf die Oberfläche oder in den Raum;
wobei in Schritt a):
- die wenigstens eine organische Verbindung ausgewählt ist aus der Gruppe bestehend aus: Azulen, dem ätherischen Öl von Thymian, dem ätherischen Öl von Nelken, einem Harz, das von dem Baum *Boswellia* erhalten wird, Derivaten der obigen organischen Verbindungen und Gemischen der obigen organischen Verbindungen;
und wobei in Schritt a):
- das bereitgestellte Fluid eine organische Verbindung in Wasser umfasst, wobei die organische Verbindung in einer Menge von 5 bis 50 Gew.-%, vorzugsweise 10 bis 30 Gew.-%, des Fluids vorhanden ist;
**dadurch gekennzeichnet, dass** in Schritt a) die wenigstens eine organische Verbindung in Kalkwasser oder in einem Gemisch aus einem nichtpolaren Medium und Kalkwasser bereitgestellt wird und wobei nach Schritt b) und vor Schritt c) die Fluidpartikel des Aerosols, deren mittlere Partikelgröße 1 Mikrometer oder mehr beträgt, von dem Aerosol abgeschieden werden.

2. Desinfektionsverfahren nach Anspruch 1, wobei die abgeschiedenen Fluidpartikel des Aerosols zu dem Fluid zurückgeführt werden, das in Schritt b) verwendet wird.

3. Desinfektionsverfahren nach einem der vorhergehenden Ansprüche, wobei in Schritt a):
- die wenigstens eine organische Verbindung in einem Gemisch aus einem nichtpolaren Medium und Kalkwasser bereitgestellt wird, wobei das nichtpolare Medium ein Öl, vorzugsweise Olivenöl, ist.

4. Desinfektionsverfahren nach einem der vorhergehenden Ansprüche, wobei in Schritt b):
- ein Gemisch aus dem Fluid und dem Gas unter Druck durch eine Düse (12) geleitet wird, sodass ein Aerosolsprühnebel aus der Düse (12) abgegeben wird.

5. Desinfektionsverfahren nach einem der Ansprüche 1 bis 3, wobei in Schritt b):
- das Gas unter Druck durch eine Düse (12) geleitet wird und während des Durchgangs durch die Düse (12) das Fluid dem Gas beigemischt wird, sodass ein Aerosolsprühnebel aus der Düse (12) abgegeben wird.

6. Desinfektionsverfahren nach einem der vorhergehenden Ansprüche 4 oder 5, wobei nach Schritt b) und vor Schritt c) die Fluidpartikel des Aerosols, deren mittlere Partikelgröße 1 Mikrometer oder mehr beträgt, von dem Aerosol abgeschieden werden, indem der Aerosolsprühnebel in eine Mischkammer (27) geleitet wird, in welcher der Sprühnebel durch Ablenkflächen abgelenkt wird und wobei das Aerosol aus dieser Mischkammer (27) durch einen Auslass (7) abgegeben wird, um Schritt c) auszuführen.

7. Desinfektionsverfahren nach Anspruch 6, wobei die Mischkammer (27) und die Düse (12) in Form einer Kartusche (1) zusammengebaut sind, die ferner ein Reservoir für das Fluid umfasst, das in fluidischer Verbindung mit der Düse (12) und der Mischkammer (27) steht.

8. Desinfektionsverfahren nach Anspruch 7, wobei die Kartusche (1) einen Einlass (5) zum Zuführen von Fluid von einer Quelle außerhalb der Kartusche (1) zu dem Reservoir umfasst.

9. Aerosol aus in einem Gas suspendierten Fluidpartikeln, wobei die Fluidpartikel eine Desinfektionsfluid-Zusammensetzung aufweisen, umfassend:
- wenigstens eine organische Verbindung, die aus natürlichen Substanzen erhältlich ist, wobei die organische Verbindung in einem polaren Medium oder in einem Gemisch aus nichtpolaren und polaren Medien vorhanden ist,
- und wobei die wenigstens eine organische Verbindung ausgewählt ist aus der Gruppe bestehend aus: Azulen, dem ätherischen Öl von Thymian, dem ätherischen Öl von Nelken, einem Harz, das von dem Baum *Boswellia* erhalten wird, Derivaten der obigen organischen Verbindungen und Gemischen der obigen organischen Verbindungen, wobei die organische Verbindung in einer Menge von 5 bis 50 Gew.-%, vorzugsweise 10 bis 30 Gew.-%, der Fluidzusammensetzung vorhanden ist;
**dadurch gekennzeichnet, dass** die wenigstens eine organische Verbindung in Kalkwasser oder in einem Gemisch aus einem nichtpolaren Medium und Kalkwasser bereitgestellt ist und wobei die Fluidpartikel eine mittlere Partikelgröße von unter 1 Mikrometer aufweisen.

10. Aerosol nach Anspruch 9, wobei
- die wenigstens eine organische Verbindung in einem Gemisch aus einem nichtpolaren Medium und Kalkwasser bereitgestellt ist, wobei das nichtpolare Medium ein Öl, vorzugsweise Olivenöl, ist.

## Revendications

1. Procédé de désinfection pour désinfecter une salle ou une surface, comprenant les étapes consistant à :
a) fournir un fluide comprenant au moins un composé organique pouvant être obtenu à partir de substances naturelles, dans lequel le composé organique est prévu dans un milieu polaire ou dans un mélange de milieux non polaire et polaire ;
b) mélanger le fluide avec un gaz de sorte que des particules de fluide sont mises en suspension dans le gaz, et un aérosol de particules de fluide est formé ;
c) diriger un écoulement de l'aérosol formé dans l'étape b) sur ladite surface ou dans ladite salle ;
dans lequel dans l'étape a) :
- l'au moins un composé organique est choisi dans le groupe constitué par : l'azulène, l'huile essentielle de thym, l'huile essentielle de clous de girofle, une résine obtenue à partir de l'arbre *Boswellia,* des dérivés des composés organiques ci-dessus, et des mélanges des composés organiques ci-dessus ;
et dans lequel dans l'étape a) :
- le fluide prévu comprend un composé organique dans de l'eau, le composé organique étant présent dans une quantité de 5 à 50 % en poids, de préférence 10 à 30 % en poids, du fluide ;
**caractérisé en ce que** dans l'étape a) l'au moins un composé organique est prévu dans de l'eau de chaux ou dans un mélange d'un milieu non polaire et d'eau de chaux et dans lequel à la suite de l'étape b) et avant l'étape c), les particules de fluide de l'aérosol dont la taille moyenne de particule est de 1 micromètre ou plus, sont séparées de l'aérosol.

2. Procédé de désinfection selon la revendication 1, dans lequel des particules de fluide séparées de l'aérosol sont renvoyées au fluide qui est utilisé dans l'étape b).

3. Procédé de désinfection selon l'une quelconque des revendications précédentes, dans lequel dans l'étape a) :
- l'au moins un composé organique est prévu dans un mélange d'un milieu non polaire et d'eau de chaux, dans lequel le milieu non polaire est une huile, de préférence de l'huile d'olive.

4. Procédé de désinfection selon l'une quelconque des revendications précédentes, dans lequel dans l'étape b) :
- un mélange du fluide et du gaz est mené sous pression à travers une buse (12), de sorte qu'une pulvérisation d'aérosol est déchargée depuis la buse (12).

5. Procédé de désinfection selon l'une quelconque des revendications 1 à 3, dans lequel dans l'étape b) :
- le gaz est mené sous pression à travers une buse (12) et le fluide est mélangé au gaz pendant le passage à travers la buse (12), de sorte qu'une pulvérisation d'aérosol est déchargée depuis la buse (12).

6. Procédé de désinfection selon l'une quelconque des revendications 4 ou 5 précédentes, dans lequel à la suite de l'étape b) et avant l'étape c), les particules de fluide de l'aérosol dont la taille moyenne de particule est de 1 micromètre ou plus, sont séparées de l'aérosol en dirigeant la pulvérisation d'aérosol dans une chambre de mélange (27) dans laquelle la pulvérisation est déviée par des surfaces de déviation, et la chambre de mélange (27) à partir de laquelle l'aérosol est déchargé via une sortie (7) en vue de la réalisation de l'étape c).

7. Procédé de désinfection selon la revendication 6, dans lequel la chambre de mélange (27) et la buse (12) sont assemblées sous la forme d'une cartouche (1) qui comprend en outre un réservoir pour le fluide qui est en communication fluidique avec la buse (12) et la chambre de mélange (27).

8. Procédé de désinfection selon la revendication 7, dans lequel la cartouche (1) comprend une entrée (5) pour fournir du fluide au réservoir à partir d'une source à l'extérieur de la cartouche (1).

9. Aérosol de particules de fluide mises en suspension dans un gaz, les particules de fluide ayant une composition de fluide désinfectant, comprenant :
- au moins un composé organique pouvant être obtenu à partir de substances naturelles, lequel composé organique est présent dans un milieu polaire ou dans un mélange de milieux non polaire et polaire,
- et dans lequel l'au moins un composé organique est choisi dans le groupe constitué par : l'azulène, l'huile essentielle de thym, l'huile essentielle de clous de girofle, une résine obtenue à partir de l'arbre *Boswellia,* des dérivés des composés organiques ci-dessus, et des mélanges des composés organiques ci-dessus, dans lequel le composé organique est présent dans une quantité de 5 à 50 % en poids, de préférence 10 à 30 % en poids, de la composition de fluide,
**caractérisé en ce que** l'au moins un composé organique est prévu dans de l'eau de chaux ou dans un mélange d'un milieu non polaire et d'eau de chaux et dans lequel les particules de fluide ont une taille moyenne de particule inférieure à 1 micromètre.

10. Aérosol selon la revendication 9, dans lequel
- l'au moins un composé organique est prévu dans un mélange d'un milieu non polaire et d'eau de chaux, dans lequel le milieu non polaire est une huile, de préférence l'huile d'olive.
